# EUROPEAN PATENT APPLICATION

(11) **EP 4 213 152 A1**
(43) Date of publication of application: **19.07.2023**
(21) Application number: 20952323.2
(22) Date of filing: 29.12.2020
(51) Int. Cl.: G16B 25/10

(54) **METHOD FOR ESTABLISHING GENOMIC SCAR MODEL**

(30) Priority: 07.09.2020 CN 202010932026
(71) Applicant: Amoy Diagnostics Co., Ltd, Xiamen, Fujian 361027 (CN)
(72) Inventor: YANG, Shuang, Xiamen, Fujian 361027 (CN); DONG, Hua, Xiamen, Fujian 361027 (CN); CHEN, Xuejun, Xiamen, Fujian 361027 (CN); HUANG, Hongwei, Xiamen, Fujian 361027 (CN); ZHENG, Fangke, Xiamen, Fujian 361027 (CN); ZHENG, Limou, Xiamen, Fujian 361027 (CN)
(74) Representative: Hoppe, Georg Johannes
(86) International application number: PCT/CN2020/140801
(87) International publication number: WO 2022/048082

(57) **Abstract**

A method for establishing a genomic scar model and the use thereof, the method comprising: (1) collecting known positive samples and negative samples to form a training set; (2) analyzing the condition of CNVs in the training set and determining the types and the corresponding number of CNVs; (3) determining BRCAness positive events and BRCAness negative events; and (4) performing training according to the BRCAness positive events and BRCAness negative events in the training set by means of a machine learning method to obtain the weights of CNVs of different types determined in step (2), and then accumulating the weights of CNVs of different types to obtain a genomic scar model for calculating a GSS. By means of the method, the BRCAness status of samples under test can be accurately predicted and an interpretation result can be directly provided according to a genomic scar score.

## Description

### FIELD OF THE DISCLOSURE

The present disclosure relates to gene test technology, and specifically relates to a constructing method of a genomic scar model.

### BACKGROUND OF THE DISCLOSURE

Homologous Recombination Repair (HRR) is an important repair method for DNA double-strand damage and is commonly used in cells to precisely repair harmful breaks in double-stranded DNA. HRR is a complex signaling pathway involving multiple steps, wherein breast cancer susceptibility genes (BRCA1/2) are important related genes for homologous recombination functions. BRCA genes can mutate to cause BRCA1 and BRCA2 proteins to lose functions so as to cause abnormal functions of the HRR, which is usually called Homologous Recombination Deficiency (HRD). As a tumor-hastening event, HRD widely exists in breast cancers, ovarian cancers, prostate cancers, and pancreatic cancers. Tumors with BRCA1/2 variations or abnormal expressions are usually sensitive to platinum chemotherapy drugs and poly [ADP-ribose] polymerase inhibitors (PARPi). Therefore, a test for BRCA1/2 gene variations plays a prominent role for clinical classification and medication guidance of these diseases.

However, when research is deeply involved, the test for the BRCA gene variations is gradually unable to meet the existing clinical needs, drug-effective populations accumulated by the test for the BRCA gene variations are relatively low, and some populations that would benefit from therapy will be missed. For example, in triple-negative breast cancer, 20% of patients have the BRCA gene variations, while an overall response rate of patient populations to platinum drugs is about 30%. At the same time, in high-grade serous ovarian cancer, 30% of patients have the BRCA gene variations, while an overall response rate of patient populations to PARPi drugs is about 50%, indicating that some patients negative to the BRCA test still respond to the platinum or the PARPi, so the BRCA test will miss some populations that would benefit from the therapy. The main reasons for missing are: first, the test for the BRCA gene variations is relatively limited. HRR function-related genes are multiple, and BRCA1/2 are two having higher variation frequencies thereof. From an analysis of an action mechanism of drugs, the HRR genes that can at least synthesize lethal effects with the platinum and the PARPi are worthy of attention (collectively called as BRCAness events), for example, results of a PROfound clinical research show that ATM deficiency of the HRR-related genes has an effect in a therapy of prostate cancer using olaparib of the PRAPi, so more and more clinical trials have shifted from focusing on the BRCA to other HRR-related genes. Second, the test for the BRCA gene variations cannot cover all types of genomic abnormalities causing functional loss of the HRR. In addition to the gene variations, methylation of a promoter region of the BCRA1 and loss of heterozygosity (LoH) within a region of the BRCA genes are also main conditions causing functional deficiencies of the HRR. Finally, interpretation of results of the test for the BRCA gene variations is complicated and easy to miss, and an entry for clinical application is high. At present, many authoritative organizations, such as the American College of Medical Genetics and Genomics (ACMG) and the European Molecular Genetics Quality Network (EMQN), have launched best practice guidelines for molecular genetic analysis of hereditary breast/ovarian cancers. Classification of BRCA variations are as follows: pathogenetic, possible pathogenetic, unidentified effects, possible benign, and benign, a grade of cited evidence in different guidelines is slightly different, which will cause great obstacles for the clinical application.

For the aforementioned reasons, needs for novel clinical molecular targets configured to simply and quantitatively evaluate the deficiencies of the homologous recombination repair of the cells are extremely urgent. Molecular targets with characteristics of downstream genomic variations (which comprises variations, copy number variation (CNV), and abnormal gene expression) caused by the deficiencies of the HRR are searched as a current main research direction. In 2009, Olafur et al. found that variation characteristics of the CNV are closely related to BRCAness. In 2012, Abkevich et al. found that a number of LoH in the whole genome was significantly related to the BRCAness events. In the same year, Popova et al. found that large-scale state transitions (LST) in the genes were related to inactivation of the BRCA1/2 genes, and Birkbak et al. also found that telomeric allelic imbalance (TAI) was related to the BRCAness events in triple-negative breast cancer and is significantly enriched in populations who are sensitive to the platinum therapy. In 2016, Myriad, Inc. in the United States quantitatively calculated HRD scores by using statistics of a number of occurrences of the LoH, the LST, and the TAI of the whole genome, the statistical index can accurately predict the BRCAness events and can be also used to effectively accumulate sensitive patients for platinum and PARPi therapies. Compared with a single test of the BRCA genes, the HRD scores can be used to screen 40% supplemental benefit-potential patients. In addition, in 2017, Davies et al. found that single-base variation patterns, long and short-scales segment rearrangement patterns, and patterns of insertions and deletions of the whole genome are closely related to the deficiencies of the HRR, these patterns are combined with the HRD scores, and the BRCAness events can be accurately predicted by Logistic regression. However, the aforementioned methods have respective limitations. For example, the HRD scores simply sum the LoH, the LST and the TAI in general, the TAI and the LoH are actually overlapped in some cases, resulting in double counting. In addition, some CNV types are also not taken into account by the HRD scores. Although it is completely taken into account by using Davies' model, the model requires whole-genomic sequencing to count patterns of various variation types, which leads to extremely expensive test costs.

### BRIEF SUMMARY OF THE DISCLOSURE

An objective of the present disclosure is to provide a constructing method of a genomic scar model to solve deficiencies of the existing techniques.

Another objective of the present disclosure is to provide a method for applying the genomic scar model constructed by the aforementioned constructing method.

A technical solution of the present disclosure is as follows:
A constructing method of a genomic scar model, it comprises:
(1) collecting known positive samples and negative samples to form a training set;
(2) analyzing a status of CNV in the training set to determine types and corresponding quantities of CNV;
(3) determining BRCAness positive events and BRCAness negative events;
(4) training to obtain weights of different types of the CNV determined in the step 2 through a machine learning method according to the BRCAness positive events and the BRCAness negative events in the training set, and then totalizing the weights of the different types of the CNV to obtain the genomic scar model for calculating GSS (genomic scar score);
(5) collecting additional known positive samples and negative samples to form a test set, and obtaining types and corresponding quantities of CNV in the test set according to the step 2; and
(6) substituting results obtained in step 5 into the genomic scar model obtained in the step 4 to calculate GSS of the test set, and verifying the genomic scar model based on a score of the GSS, and completing.

In a preferred embodiment of the present disclosure, in the step 2: sequencing and analyzing the training set obtained in the step 1; calculating the status of the CNV in results of the sequencing and the analyzing; joining adjacent regions with a same CNV into fragments to avoid double counting; and determining the types and the corresponding quantities of the CNV.

Further preferably, the sequencing and the analyzing is based on whole genome, whole exome, target capture sequencing, or a chip of CNV.

In a preferred embodiment of the present disclosure, the BRCAness positive events comprise: in any one of BRCA1/2, a pathogenic or suspected pathogenic variation occurs in one allele, and loss of heterozygosity occurs in another allele; in any one of BRCA1/2, two pathogenic or suspected pathogenic variations occur; and in BCRA1, loss of heterozygosity occurs in one allele, and methylation occurs in a promoter region of another allele.

The BRCAness positive events further comprise homologous recombination repair related genes other than BRCA1/2 genes that have corresponding gene variations, homozygous deletions, and expression silencing configured to cause genome instability related events.

In a preferred embodiment of the present disclosure, the BRCAness negative events comprises:HRR-related genes are wild-type, and no loss of heterozygosity occurs in corresponding alleles or no methylation occurs in a promoter region thereof.

In a preferred embodiment of the present disclosure, the types of the CNV in the step 2 are determined according to lengths of variation segments, types of the variation segments, and genomic location of the variation segments.

In a preferred embodiment of the present disclosure, the lengths of the variation segments are divided into a short segment of 5-10 M, a medium segment of greater than 10 M and less than or equal to 15 M, and a long segment of greater than 15 M.

In a preferred embodiment of the present disclosure, the length of the variation segments are divided into continuous variables, and the continuous variables comprises 5, 6, 7, 8, 9...30 M lengths of the CNV segments.

In a preferred embodiment of the present disclosure, the types of the variation segments comprise loss of heterozygosity, Allele specific CNV, and Balance CNV.

In a preferred embodiment of the present disclosure, a location of the variation segments on a genome comprises the variation segments located on a side of telomere, the variation segments located on an inner side of a centromeric region, and the variation segments located on positions other than the side of the telomere and the inner side of the centromere region.

A first one of other technical solutions of the present disclosure is as follows:
A method for applying the genomic scar model constructed by the constructing method to accumulate populations with HRR-related variations.

A second one of other technical solutions of the present disclosure is as follows:
A method for applying the genomic scar model constructed by the constructing method to accumulate sensitive populations to platinum drugs.

A third one of other technical solutions of the present disclosure is as follows:
A method for applying the genomic scar model constructed by the constructing method to accumulate sensitive populations to PARPi drugs.

According to the present disclosure:
1. Compared with the test for the BRCA gene variations, the present disclosure can accurately predict BRCAness status of the sample to be tested without testing methylation of a promoter region of the BRCA1 and loss of heterozygosity of the BRCA genes. In addition, compared with complicated interpretation of the BRCA variations, the present disclosure can directly provide interpretation results based on genomic scar scores.
2. Compared with the test for the BRCA gene variations, the present disclosure can accumulate patients with HRR-related gene variations.
3. Compared with the test for the BRCA gene variations, the present disclosure can accumulate more patients sensitive to platinum drugs.
4. Compared with the test for the BRCA gene variations, the present disclosure can accumulate more patients sensitive to PARPi drugs.

### BRIEF DESCRIPTION OF THE DRAWINGS

FIG. 1 illustrates a graph of experimental results of Embodiment 3 of the present disclosure.
FIG. 2 illustrates a first graph of experimental results of Embodiment 4 of the present disclosure.
FIG. 3 illustrates a second graph of the experimental results of Embodiment 4 of the present disclosure.

### DETAILED DESCRIPTION OF THE EMBODIMENTS

The technical solution of the present disclosure will be further described below in combination with the accompanying embodiments and drawings.

### Embodiment 1

110 and 18 FFPE samples and control blood samples of ovarian cancer patients as a test sample type are respectively collected to function as a training set and a test set for constructing the genomic scar model. A Homologous Recombination Deficiency (HRD) test kit of Amoy Diagnostics Co., Ltd. is then used for library construction and capture. The kit contains 35 HRR-related genes and 70,000 snp sites functioning as capture regions. Captured and enriched DNA is finally sequenced on an Illumina Novaseq sequencer.

Raw data is compared with the human genome reference sequence (a version number is hg19) through BWA (Li H. and Durbin R. 2009). BAM files after comparison are generated to function as input files for variation and copy number variation. A test of the variation uses the Varscan process (Koboldt, D.2012), and a chain-specific copy number variation uses the sequenza (Favero F.2015) process.

Confirmation of BRCAness samples. Most common BRCAness events at the moment are selected as a positive sample tag and specifically comprise: a. in any one gene of BRCA1/2, a pathogenic or suspected pathogenic variation occurs in one allele, and loss of heterozygosity occurs in another allele; b. in any one gene of BRCA1/2, two pathogenic or suspected pathogenic variations occur, that is, functional loss; c. in BCRA1, the loss of heterozygosity occurs in one allele, and methylation occurs in a promoter region of another allele, wherein like-methylation of a promoter region of BCRA1 is obtained by a pyrosequencing technology.

Confirmation of BRCAness-negative samples, HRR-related mutant genes are wild-type, and the LoH does not occur in a corresponding gene. HRD test reagents of Amoy Diagnostics comprise the following HRR-related genes: ATM, FAM175A, FANCI, NBN, RAD51C, ATR, FANCA, FANCL, PALB2, RAD51D, ATRX, FANCC, FANCM, RAD50, RAD52, BAP1, FANCD2, KMT2D, RAD51 , RAD54L, BARD1, FANCE, MDC1, RAD51B, SLX4, BLM, FANCF, MRE11A, WRN, XRCC2, BRCA1, FANCG, BRCA2, BRIP1, and EMSY.

Segments of the copy number variation in the embodiment are classified according to lengths of the variation and comprise short segments (5-10 M), medium segments (greater than 10 and less than or equal to 15 M), and long segments (>15 M). The segments of the copy number variation can also be classified according to variation types, comprising Loss of heterozygosity (LOH), Allele specific CNV (ASCNV), and Balance CNV (BCNV). The segments of the copy number variation can also be classified according to genomic location thereof and comprises the variation segments located on a side of telomere, the segments of the variation segments located on an inner side of a centromere region and other remaining regions. The segments of the copy number variation are finally classified into 27 types (namely, length types × variation types × genomic location types is equal to 27).

68 BRCAness samples and 42 negative samples in the training set and 10 BRCAness samples and 8 negative samples in the test set are processed according to the abovementioned method. In order to prevent over-fitting during a training process, only types in which a number of occurrences of the segments of the copy number variation in training samples are larger than a number of samples in the training set are reserved. A weight of the types of the segments of the copy number variation is then trained using Logistic regression according to the types of the BRCAness samples to construct a genomic scar model. A sample to be tested is calculated by the genomic scar model, a sample with a GSS score less than 0.5 is determined as a BRCAness negative sample, and a sample with a GSS score greater than 0.5 is determined as a BRCAness positive sample.

In the test set, a GSS of the sample to be tested is calculated using the genomic scar model to determine BRCAness status of the sample to be tested. It is then compared with BRCAness status of the samples labeled in advance in the test set, wherein 10 BRCAness positive samples are accurately determined as positive by the genomic scar model, and the 8 BRCAness negative samples are accurately determined as negative by the genomic scar model. That is, the GSS of the genomic scar model can accurately predict the BRCAness status of the sample, a sensitivity is 100%, a specificity is 100%, and an accuracy is 100%.

### Embodiment 2

191 FFPE samples and control blood samples of ovarian cancer patients as a test sample type are collected. A Homologous Recombination Deficiency (HRD) test kit of Amoy Diagnostics Co., Ltd. is then used for library construction and capture, a sequence is determined on the Illumina Novaseq sequencer, and the GSS of the sample to be tested is then calculated using the genomic scar model trained in Embodiment 1.

A statistic of relationships between a GSS high-score group and HRR variation populations is shown in the table described below.

| | No HRR-related genetic variation group | HRR-related genetic variation group |
|---|---|---|
| GSS low-score group | 52 | 19 |
| The GSS high-score group | 56 | 64 |

Through a hypergeometric distribution test, the GSS high-score group significantly accumulates patients with HRR-related genetic variations, P=0.0003. In addition, compared with the HRR-related genes, the GSS of the genomic scar model obtained in Embodiment 1 can accumulate more patients with genomic instability.

### Embodiment 3

44 FFPE samples and control blood samples of ovarian cancer patients as a test sample type are collected. A first therapy after surgery for these patients is platinum chemotherapy. A Homologous Recombination Deficiency (HRD) test kit of Amoy Diagnostics Co., Ltd. is then used for library construction and capture, a sequence is determined on the Illumina Novaseq sequencer, and the GSS of the sample to be tested is then calculated using the genomic scar model trained in Embodiment 1.

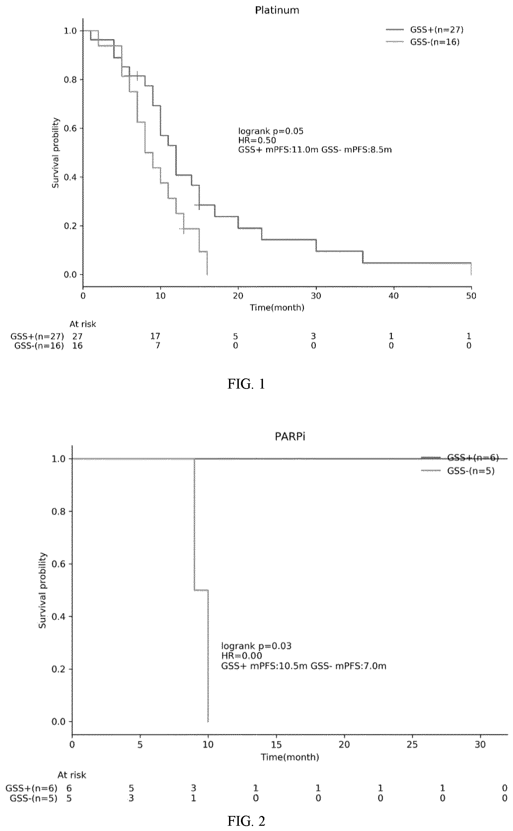

A function of the GSS in accumulating platinum drug sensitive populations is evaluated by comparing progression-free survivals (PFS) of the GSS high-score group and the GSS low-score group of the patients, and the results are shown in FIG. 1. Referring to FIG. 1, GSS status, namely GSS high score (GSS+) and GSS low score (GSS-) branch groups, show that the progression-free survivals of the patients have a significant difference, the PFS of the patients in the GSS high-score group using a platinum therapy is significantly longer (P=0.05), wherein a median PFS of the patients of the GSS high-score group is equal to 11 months, and a median PFS of the patients of the GSS low-score group is equal to 8.5 months.

### Embodiment 4

14 and 20 FFPE samples and control blood samples of ovarian cancer patients as a test sample type are collected, and these patients respectively receive a first-line maintenance therapy and a rear-line therapy, namely PARPi. A Homologous Recombination Deficiency (HRD) test kit of Amoy Diagnostics Co., Ltd. is then used for library construction and capture, a sequence is determined on the Illumina Novaseq sequencer, and the GSS of the sample to be tested is then calculated using the genomic scar model trained in Embodiment 1.

In the ovarian cancer patients who receive the first-line maintenance therapy using the PARPi, referring to FIG. 2, with respect to GSS high score (GSS+) and GSS low score (GSS-) branch groups, the progression-free survivals of the patients have a significant difference. The progression-free survivals of the patients in the GSS high-score group using the PARPi therapy is significantly longer (P=0.03), wherein a median PFS of the patients of the high-score GSS group is equal to 10.5 months, and the median PFS of patients in the low-score GSS group is equal to 7 months.

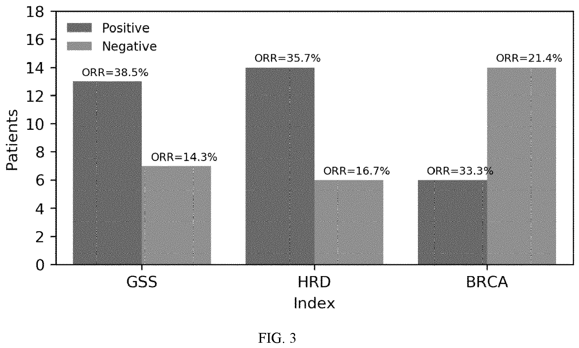

In the ovarian cancer patients with the rear-line therapy using PARPi, an objective response rate (ORR) in the GSS high-score group is 38.5% (5/13), the objective response rate is highest compared to a HRD high-score group (35.7%) and a BRCA variation group (33.3%), an objective response rate in the GSS low-score group is 14.3% (1/7), the objective response rate is the lowest compared to a low-score HRD group (16.7%) and a BRCA wild-type group (21.4%), and the results are shown in the FIG. 3. The GSS of the genomic scar model obtained in Embodiment 1 can enrich sensitive populations of PARPi drugs.

The aforementioned embodiments are merely some embodiments of the present disclosure, and the scope of the disclosure is not limited thereto. Thus, it is intended that the present disclosure cover any modifications and variations of the presently presented embodiments provided they are made without departing from the appended claims and the specification of the present disclosure.

### INDUSTRIAL APPLICABILITY

The present disclosure discloses a method for constructing a genomic scar model, it comprises: (1) collecting known positive samples and negative samples to form a training set; (2) analyzing a status of CNV in the training set to determine types and corresponding quantities of CNV; (3) determining BRCAness positive events and BRCAness negative events; (4) training to obtain weights of different types of the CNV determined in the step 2 through a machine learning method according to the BRCAness positive events and the BRCAness negative events in the training set, and then totalizing the weights of the different types of the CNV to obtain the genomic scar model for calculating GSS. The present disclosure can accurately predict BRCAness status of the sample to be tested, can directly provide interpretation results based on genomic scar scores and has good industrial applicability.

## Claims

1. A constructing method of a genomic scar model, **characterized in that** it comprises:
(1) collecting known positive samples and negative samples to form a training set;
(2) analyzing a status of CNV in the training set to determine types and corresponding quantities of CNV;
(3) determining BRCAness positive events and BRCAness negative events;
(4) training to obtain weights of different types of the CNV determined in the step 2 through a machine learning method according to the BRCAness positive events and the BRCAness negative events in the training set, and then totalizing the weights of the different types of the CNV to obtain the genomic scar model for calculating GSS;
(5) collecting additional known positive samples and negative samples to form a test set, and obtaining types and corresponding quantities of CNV in the test set according to the step 2; and
(6) substituting results obtained in step 5 into the genomic scar model obtained in the step 4 to calculate GSS of the test set, and verifying the genomic scar model based on a score of the GSS, and completing.

2. The constructing method according to claim 1, wherein:
the BRCAness positive events comprise:
in any one of BRCA1/2, a pathogenic or suspected pathogenic variation occurs in one allele, and loss of heterozygosity occurs in another allele;
in any one of BRCA1/2, two pathogenic or suspected pathogenic variations occur; and
in BCRA1, loss of heterozygosity occurs in one allele, and methylation occurs in a promoter region of another allele.

3. The constructing method according to claim 2, wherein the BRCAness positive events further comprise homologous recombination repair related genes other than BRCA1/2 genes that have corresponding gene variations, homozygous deletions, and expression silencing configured to cause genome instability related events.

4. The constructing method according to claim 1, wherein the BRCAness negative events comprises:
HRR-related genes are wild-type, and
no loss of heterozygosity occurs in corresponding alleles or no methylation occurs in a promoter region thereof.

5. The constructing method according to claim 1, wherein the types of the CNV in the step 2 are determined according to lengths of variation segments, types of the variation segments, and genomic location of the variation segments.

6. The constructing method according to claim 5, wherein the lengths of the variation segments are divided into a short segment of 5-10 M, a medium segment of greater than 10 M and less than or equal to 15 M, and a long segment of greater than 15 M.

7. The constructing method according to claim 5, wherein the length of the variation segments are divided into continuous variables.

8. The constructing method according to claim 5, wherein the types of the variation segments comprise loss of heterozygosity, Allele specific CNV, and Balance CNV.

9. The constructing method according to claim 5, wherein a location of the variation segments on a genome comprises the variation segments located on a side of telomere, the variation segments located on an inner side of a centromeric region, and the variation segments located on positions other than the side of the telomere and the inner side of the centromere region.

10. The constructing method according to claim 1, wherein:
training the weights of the types of the CNV using logistic regression according to the BRCAness types of the samples to construct the genomic scar model, and calculating samples to be tested by the genomic scar model to determine a sample with a GSS score less than 0.5 as a BRCAness negative sample and a sample with a GSS score greater than 0.5 as a BRCAness positive sample.

11. A method for applying the genomic scar model constructed by the constructing method of any one or more of claims 1-10 to accumulate populations with HRR-related variations.

12. A method for applying the genomic scar model constructed by the constructing method of any one or more of claims 1-10 to accumulate sensitive populations to platinum drugs.

13. A method for applying the genomic scar model constructed by the constructing method of any one or more of claims 1-10 to accumulate sensitive populations to PARPi drugs.
